Europäisches Patentamt
European Patent Office
Office européen des brevets

(19)

(11) Publication number: 0 387 661 A2

(12) EUROPEAN PATENT APPLICATION

(21) Application number: 90104235.8

(51) Int. Cl.5: C07H 15/252, C07H 19/02, A61K 31/70

(22) Date of filing: 06.03.90

(30) Priority: 13.03.89 GB 8905668

(43) Date of publication of application: 19.09.90 Bulletin 90/38

(84) Designated Contracting States: AT BE CH DE DK ES FR GB GR IT LI NL SE

(71) Applicant: FARMITALIA CARLO ERBA S.r.L.
Via Carlo Imbonati 24
I-20159 Milano(IT)

(72) Inventor: Suarato, Antonino
Via Degli Imbriani, 39
I-20138 Milan(IT)
Inventor: Angelucci, Francesco
Via Washington, 106
I-20100 Milan(IT)
Inventor: Bargiotti, Alberto
Via Donati, 8
I-20100 Milan(IT)
Inventor: Grandi, Maria
Piazza 5 Giornate, 6
I-20100 Milan(IT)
Inventor: Pezzoni, Gabriella
Via Pomezia 10/a
I-20100 Milan(IT)

(74) Representative: Giambrocono, Alfonso, Dr. Ing. et al
Ing. A. Giambrocono & C. S.r.l. Via Rosolino Pilo 19/B
I-20129 Milano(IT)

# (54) New 3'-(4-morpholinyl)- and 3'-(2-methoxy-4-morpholinyl)-anthracycline derivatives.

(57) Anthracycline glycosides of general formula I:

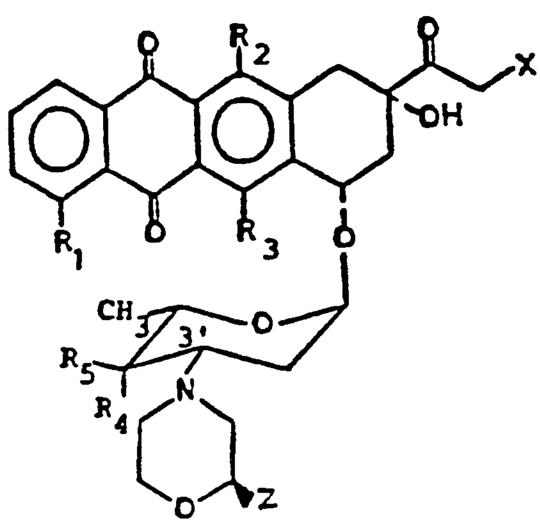

wherein Z is hydrogen or methoxy;
X represents hydrogen or hydroxy;
$R_1$ represents hydrogen, fluorine, hydroxy, methoxy or amino;
$R_2$ and $R_3$ both represent hydroxy or one of $R_2$ and $R_3$ is hydrogen, nitro or amino and the other is

EP 0 387 661 A2

hydroxy; and
$R_4$ and $R_5$ represent hydrogen or one of $R_4$ and $R_5$ is hydrogen and the other is hydroxy;
and pharmaceutically acceptable salts thereof; with the provisos that:
(i) when $R_2$ and $R_3$ are both hydroxy and Z is hydrogen then $R_1$ represents hydroxy, fluorine or amino, and
(ii) when both Z and $R_1$ are methoxy and $R_2$ and $R_3$ are both hydroxy then $R_5$ is hydroxy or both $R_4$ and $R_5$ are hydrogen.

# NEW 3'-(4-MORPHOLINYL)- AND 3'-(2-METHOXY-4-MORPHOLINYL)ANTHRACYCLINE DERIVATIVES

The invention relates to anthracycline glycosides, to processes for their preparation and to pharmaceutical compositions containing them.

The invention provides anthracycline glycosides having the general formula I:

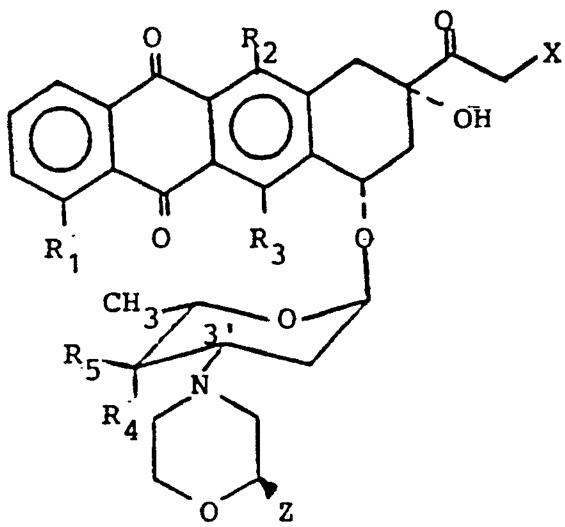

wherein Z is hydrogen or methoxy;
X represents hydrogen or hydroxy;
$R_1$ represents hydrogen, fluorine, hydroxy, methoxy, amino;
$R_2$ and $R_3$ both represent hydroxy or one of $R_2$ and $R_3$ is hydrogen, nitro or amino and the other is hydroxy; and
$R_4$ and $R_5$ both represent hydrogen or one of $R_4$ and $R_5$ is hydrogen and the other is hydroxy;
and pharmacuetically acceptable salts thereof; with the provisos that

(i) when $R_2$ and $R_3$ are both hydroxy and Z is hydrogen then $R_1$ represents hydroxy, fluorine or amino, and

(ii) when Z and $R_1$ are methoxy and $R_2$ and $R_3$ are hydroxy then $R_5$ is hydroxy or both $R_4$ and $R_5$ are hydrogen.

Preferred, pharmaceutically acceptable acid addition salts are the hydrochloride salts. The preferred anthracycline glycosides of general formula I include:

a) : 4-demethyl-6-deoxy-3'-deamino-3'-(4-morpholinyl) doxorubicin
($X=OH$, $R_1=R_2=R_4=OH$, $R_3=R_5=H$, $Z=H$)

b) : 4-demethoxy-4-amino-3'-deamino-3'-(4-morpholinyl)-daunorubicin
($X=H$, $R_1=NH_2$, $R_2=R_3=R_4=OH$, $R_5=H$, $Z=H$)

c) : 4-demethoxy-4-amino-3'-deamino-3'-(4-morpholinyl)-4'-epi-daunorubicin
($X=H$, $R_1=NH_2$, $R_2=R_3=R_5=OH$, $R_4=H$, $Z=H$)

d) : 4-demethoxy-4-fluoro-3'-deamino-3'-(4-morpholinyl)-daunorubicin
($X=H$, $R_1=F$, $R_2=R_3=R_4=OH$, $R_5=H$, $Z=H$)

e) : 4-demethoxy-4-fluoro-3'-deamino-3'-(4-morpholinyl)-4'-epi-daunorubicin
($X=H$, $R_1=F$, $R_2=R_3=R_5=OH$, $R_4=H$, $Z=H$)

f) : 4-demethoxy-11-deoxy-11-nitro-3'-deamino-3'-(4-morpholinyl)-daunorubicin
($X=H$, $R_1=R_5=H$, $R_2=NO_2$, $R_3=R_4=OH$, $Z=H$)

g) : 4-demethoxy-6-deoxy-6-nitro-3'-deamino-3'-(4- morpholinyl)-daunorubicin
($X=H$, $R_1=R_5=H$, $R_3=NO_2$, $R_2=R_4=OH$, $Z=H$)

h) : 4-demethoxy-11-deoxy-11-amino-3'-deamino-3'-(4-morpholinyl)-daunorubicin
($X=H$, $R_1=R_5=H$, $R_2=NH_2$, $R_3=R_4=OH$, $Z=H$)

i) : 4-demethoxy-6-deoxy-6-amino-3'-deamino-3'-(4-morpholinyl)-daunorubicin
($X=H$, $R_1=R_5=H$, $R_3=NH_2$, $R_2=R_4=OH$, $Z=H$)

j) : 3'-deamino-3'-(2-methoxy-4-morpholinyl)-4'-epi-doxorubicin
(X = OH, $R_1$ = $OCH_3$, $R_2$ = $R_3$ = OH, $R_4$ = H, $R_5$ = OH, Z = $OCH_3$)
k) : 3'-deamino-3'-(2-methoxy-4-morpholinyl)-4'-deoxydoxorubicin
(X = OH, $R_1$ = $OCH_3$, $R_2$ = $R_3$ = OH, $R_4$ = $R_5$ = H, Z = $OCH_3$)
l) : 4-demethoxy-3'-deamino-3'-(2-methoxy-4-morpholinyl)-daunorubicin
(X = H, $R_1$ = $R_5$ = H, $R_2$ = $R_3$ = $R_4$ = OH, Z = $OCH_3$)
m) : 4-demethyl-3'-deamino-3'-(2-methoxy-4-morpholinyl)-daunorubicin
(X = H, $R_1$ = $R_2$ = $R_3$ = $R_4$ = OH, $R_5$ = H, Z = $OCH_3$)
n) : 4-demethyl-6-deoxy-3'-deamino-3'-(2-methoxy-4-morpholinyl)-doxorubicin
(X = OH, $R_1$ = $R_2$ = $R_4$ = OH, $R_3$ = $R_5$ = H, Z = $OCH_3$)
o) : 4-demethoxy-4-amino-3'-deamino-3'-(2-methoxy-4-morpholinyl)-daunorubicin
(X = H, $R_1$ = $NH_2$, $R_2$ = $R_3$ = $R_4$ = OH, $R_5$ = H, Z = $OCH_3$)
p) : 4-demethoxy-11-deoxy-11-amino-3'-deamino-3'-(2- methoxy-4-morpholinyl)-daunorubicin
(X = H, $R_1$ = $R_5$ = H, $R_2$ = $NH_2$, $R_3$ = $R_4$ = OH, Z = $OCH_3$)
q) : 4-demethoxy-6-deoxy-6-amino-3'-deamino-3'-(2-methoxy-4-morpholinyl)-daunorubicin
(X = H, $R_1$ = $R_5$ = H, $R_3$ = $NH_2$, $R_2$ = $R_4$ = OH, Z = $OCH_3$)
r) : 4-demethoxy-6-deoxy-6-amino-3'-deamino-3'-(4-morpholinyl)-doxorubicin
(X = OH, $R_1$ = $R_5$ = H, $R_3$ = $NH_2$, $R_2$ = $R_4$ = OH, Z = H)

The new anthracycline glycoside antibiotics of the invention are prepared by the formation of a morpholinyl or a methoxy-substituted morpholinyl ring at C-3' on the sugar moiety of the antitumor anthracycline glycosides of general formula II:

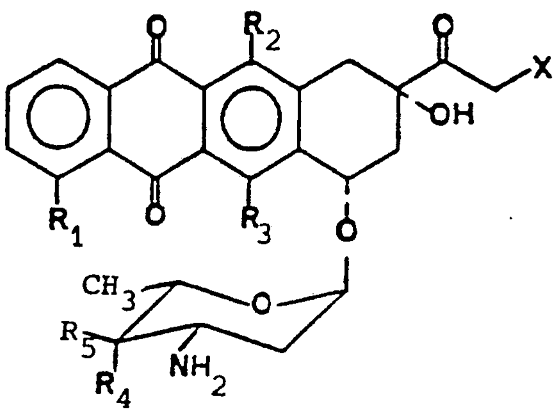

wherein $R_1$, $R_2$, $R_3$, $R_4$, $R_5$ and X are as above defined or a salt thereof, for example a pharmaceutically acceptable acid addition salt such as the hydrochloride salt. An anthracycline glycoside of formula I wherein Z = H or a pharmaceutically acceptable salt is therefore prepared by reacting an anthracycline glcoside of formula II or a salt thereof, for example a pharmaceutically acceptable acid addition salt such as the hydrochloride salt, with bis(2-iodoethyl)ether and, if desired, convering the anthracycline glycoside of formula I wherein Z is H thus obtained into a pharmaceutically acceptable salt thereof. An anthracycline glycoside of formula I where Z = $OCH_3$ or a pharmaceutically acceptable salt thereof is prepared by effecting reductive alkylation of an anthracycline glycoside of formula II or a salt thereof, for example a pharmaceutically acceptable acid addition salt such as the hydrochloride salt, using the chiral dialdehyde 1-methoxy-2,2'-oxydiacetaldehyde of formula III:

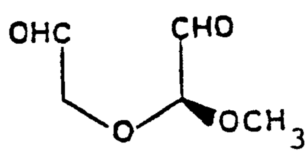

for example under the conditions reported in US-A-4672057; and, if desired, convering the resulting anthracycline glycoside of formula I wherein Z is $OCH_3$ into a pharmaceutically acceptable salt thereof.

More particularly the reaction for preparing 3'-(4-morpholino) anthracycline derivatives of formula I is

typically carried out using an excess of bis(2-iodoethyl)-ether in an anhydrous polar organic solvent, such as dimethylformamide. The reaction is generally carried out at room temperature. It can be usually completed in one day. The desired product is isolated from the reaction mixture, for example by solvent extraction. It may be purified by chromatography, for example column chromatography. It may be transformed into the hydrochloride salt, for example by treatment with methanolic hydrogen chloride.

In one embodiment, therefore, the anthracycline glycoside of formula II or salt thereof dissolved in an anhydrous polar solvent is reacted, at room temperature and for 24 hours, with an excess of bis(2-iodoethyl)ether in the presence of triethylamine; the pH of the reaction mixture, previously diluted with water, is adjusted to 7.5 using an aqueous solution of sodium hydrogen carbonate; the reaction mixture is extracted with methylene chloride; the organic solvent is removed under reduced pressure; and, after purification of the crude product on a column of silica gel using a mixture of methylene chloride and methanol (98:2 v/v) as eluting agent, the product is isolated as the hydrochloride salt by addition of a methanolic solution of anhydrous hydrogen chloride. The reductive alkylation for preparing 3′-(2-methoxy-4-morpholino) anthracycline derivatives of formula I is typically carried out using an excess of the dialdehyde III. This dialdehyde can be prepared as described in US-A-4672057. The reaction is generally carried out in a mixed aqueous polar organic medium, such as water-acetonitrile, generally at pH of about 7. A reducing agent such as an alkali metal cyanoborohydride, i.e. sodium or potassium cyanoborohydride, is generally present.

The reaction can be usually completed in two hours at room temperature. The desired product is isolated from the reaction mixture, for example by solvent extraction. It may be purified by a common chromatography, for example column chromatography. The product may be transformed into the hydrochloride salt, for example by treatment with methanolic hydrogen chloride.

In a second embodiment, therefore, the anthracycline glycoside of formula II or salt thereof, dissolved in a mixture of acetonitrile and water (1:1 by volume) is reacted with an excess of the aldehyde of formula III; after 30 minutes added to the reaction mixture, adjusted to pH 7.4, is an aqueous solution of sodium cyanoborohydride; the mixture is extracted with n-butanol; the organic solent is removed under reduced pressure; and, after purification of the crude product on a column of silica gel using a mixture of methylene chloride-methanol (97:3 v/v) as eluting agent, the desired product is isolated as the hydrochloride salt by addition of a methanolic solution of anhydrous hydrogen chloride.

The starting materials for making the new 3′-(4-morpholino) or 3′-(2-methoxy-4-morpholino) glycosides of the invention are:

- the well known 4′-epi-doxorubicin (C1: X=OH, $R_1$=$OCH_3$, $R_2$=$R_3$=$R_5$=OH, $R_4$=H), 4′-deoxy-doxorubicin (C2: X=OH, $R_1$=$OCH_3$, $R_2$=$R_3$=OH, $R_4$=$R_5$=H), 4-demethoxy-daunorubicin (C3: X=H, $R_1$=$R_5$=H, $R_2$=$R_3$=$R_4$=OH) and 4-demethyl-daunorubicin (C4: X=H, $R_1$=$R_2$=$R_3$=$R_4$=OH, $R_5$=H);
- 4-demethyl-6-deoxy-doxorubicin (C5: X=OH, $R_1$=$R_2$=$R_4$=OH, $R_3$=$R_5$=H) (US-A-4600537);
- 4-demethoxy-4-amino-daunorubicin (C6: X=H, $R_1$=$NH_2$, $R_5$=H, $R_2$=$R_3$=$R_4$=OH) and 4-demethoxy-4-amino-4′-epi-daunorubicin (C7: X=H, $R_1$=$NH_2$, $R_4$=H, $R_2$=$R_3$=$R_5$=OH) (EP-A-0288268);
- 4-demethoxy-4-fluoro-daunorubicin (C8: X=H, $R_1$=F, $R_4$=H, $R_2$=$R_3$=$R_5$=OH) and 4-demethoxy-4-fluoro=4′-epi-daunorubicin (C9: X=H, $R_1$=F, $R_4$=H, $R_2$=$R_3$=$R_5$=OH) (US-A-4697005);
- 4-demethoxy-11-deoxy-11-nitro-daunorubicin (C10: X=H, $R_1$=$R_5$=H, $R_2$=$NO_2$, $R_3$=$R_4$=OH) and 4-demethoxy-6-deoxy-6-nitro-daunorubicin (C11: X=H, $R_1$=$R_5$=H, $R_3$=$NO_2$, $R_2$=$R_4$=OH) (US-A-4684639);
- 4-demethoxy-11-deoxy-11-amino-daunorubicin (C12: X=H, $R_1$=$R_5$=H, $R_2$=$NH_2$, $R_3$=$R_4$=OH) and 4-demethoxy-11-deoxy-11-amino-doxorubicin (C13: X=OH, $R_1$=$R_5$=H, $R_2$=$NH_2$, $R_3$=$R_4$=OH) (US-A-4348388); and
- 4-demethoxy-6-deoxy-6-amino-daunorubicin (C14: X=H, $R_1$=$R_5$=H, $R_3$=$NH_2$, $R_2$=$R_4$=OH) and 4-demethoxy-6-deoxy-6-amino-doxorubicin (C15: X=OH, $R_1$=$R_5$=H, $R_3$=$NH_2$, $R_2$=$R_4$=OH) (EP-A-0254484);

and salts thereof such as the hydrochloride salts.

The invention provides pharmaceutical compositions comprising an anthracycline glycoside of formula I or a pharmaceutically acceptable salt thereof, together with a pharmaceutically acceptable diluent or carrier. Coventional carriers and diluents may be used. The composition may be formulated and administered in conventional manner.

The compounds of the invention are useful in methods of treatment of the human or animal body by therapy. They are useful as antitumor agents. A therapeutically effective amount is administered to a patient. An amount sufficient to inhibit the growth of the tumour may be administered. The tumor may be a Colon adenocarcinoma or Gross leukaemia tumor.

The biological activity of the compounds according to the invention was tested <u>in vitro</u> against LoVo (human colon adenocarcinoma) and LoVo/DX cells in comparison with doxorubicin. The results are shown in

Table 1. The compounds of the invention were also tested in vivo against P388 and P388/DX murine leukemia in comparison with doxorubicin. The results are shown in Table 2. All compounds were tested as their hydrochlorides.

Table 1

| in vitro activity | | | |
|---|---|---|---|
| Compounds | Cytotoxicity after 4h of treatment (IC50* = ng/ml) | | |
| | LoVo | LoVo/DX | R.I.** |
| Doxorubicin | 60 | 2180 | 36 |
| Ia | 35.5 | 165.4 | 4.7 |
| Ib | 13.7 | 28.5 | 2.1 |
| Ic | 36 | 48.5 | 1.3 |
| Ie | 47 | 168 | 3.6 |
| II | 0.2 | 2.3 | 11.5 |

* IC50 = concentration inhibiting by 50% colony growth
** R.I. = Resistance Index = (IC50 LoVo/DX)/(IC50 LoVo)

Table 2

| Antileukemic activity against P388 sensitive and resistant to doxorubicin | | | | |
|---|---|---|---|---|
| Compounds | P388 ( iv, iv+1) | | P388/DX Johnson (iv , iv+1) | |
| | O.D.* (mg/Kg) | %T/C** | O.D.* (mg/Kg) | %T/C** |
| Doxorubicin | 13 | 250 | 13 | 100 |
| Ia | 1.6 | 222 | 1.12 | 183 |
| Ib | 0.76 | 156 | 0.575 | 167 |
| Ic | | | 20 | 195 |
| Ie*** | 6.3 | 411 | 6.3 | 244 |
| II | 0.14 | 133 | 0.36 | 161 |
| iv means that tumor has been implanted intravenously; iv+1 means that treatment begins one day after said implantation. | | | | |

* Optimal dose: maximum tolerated dose (LD10);
** Median survival time of treated mice/median survival time of controls x 100;
*** 3 of 10 mice were considered long term survivors at the end of the experiments (90 days).

The following Examples illustrate the invention. Thin layer chromatography (TLC) measurements were made on Kieselgel plates (Merck $F_{254}$) using methylene chloride/ethanol (95/5 by volume) as eluent. "Merck" is a Trade Mark.

Example 1

4-demethyl-6-deoxy-3′-deamino-3′-(4-morpholinyl)doxorubicin (a)

To a solution of 0.15g (0.27 mmole) of 4-demethyl-6-deoxy-doxorubicin hydrochloride (C5) in 5 ml of anhydrous dimethylformamide, was added 1g (3.07 mmole) of bis(2-iodoethyl)ether and 0.075 ml (0.535 mmole) of triethylamine. The reaction mixture was stirred at room temperature for one day, then the mixture was diluted with 100 ml of water and the pH was adjusted to 7.5 with a solution of aqueous sodium hydrogen carbonate. The aqueous layer was extracted with methylene chloride three times. The organic phase was dried over anhydrous sodium sulphate and the solvent removed under reduced pressure. The residue was chromatographed on a column of silica gel using methylene chloride/methanol (98:2 v/v) as eluting system to give 70 mg (yield 42%) of the title compound (a) which was isolated as hydrochloride salt after addition with a methanolic solution of anhydrous hydrogen chloride.
m.p. 161-162° C (dec.); Rf:0.19; FD-MS: m/z 583 (M+.);
$^1$HNMR (200 MHz, DMSO) inter alia δ:
12.94 (s, 1H, 11-OH,), 12.53 (s, 1H, 4-OH), 7.9-7.7 (m, 3H, 6-H, 1-H, 2-H), 5.77 (d, J=5.7 Mz, 1H, 4′-OH), 5.42 (d , J=5.9, 7.4Hz, 1H, 7-H), 4.54 (s, 2H, $CH_2OH$), 4.0-2.7 (m, 13H, 5′-H, 4′-H, 3′-H, 10-$CH_2$, $CH_2OCH_2$, $CH_2$ $NCH_2$), 1.19 (d, J=6.4Hz, 3H, 5′-$CH_3$)

Example 2

4-demethoxy-4-amino-3′-deamino-3′-(4-morpholinyl)daunorubicin (b)

Following the procedure described in Example 1, the title compound (b) was obtained starting from 4-demethoxy-4-amino-daunorubicin (C6).
Yield 80%, m.p. 167-168° C (dec.); Rf:0.33; FD-MS: m/z 582 (M+.) $^1$HNMR (200 MHz, $CDCl_3$) inter alia δ: 13.73 (s, 1H, 6-OH), 13.40 (s, 1H, 11-OH), 7.67 (d, J=6.4Hz, 1H, 1-H), 7.49 (dd, J=6.4, 8.3Hz, 1H, 2-H), 6.97 (d, J=8.3Hz, 3-H), 6.81 (broad, 2H, $NH_2$), 5.56 (d, J=3.2Hz, 1H, 1′-H), 5.26 (dd, J=2.2, 3.8Hz, 1H, 7-H), 3.68 (m, 4H, $CH_2OCH_2$), 2.6-2.3 (m, 6H, $CH_2NCH_2$), 2.42 (s, 3H, $COCH_3$), 1.38 (d, J=6.6Hz, 3H, 5′-$CH_3$).

Example 3

4-demethoxy-4-fluoro-3′-deamino-3′-(4-morpholino)-4′-epi-daunorubicin (e)

Following the procedure described in Example 1, the title compound (e) was obtained starting from 4-demethoxy-4-fluoro-4′-epi-daunorubicin (C9).
Yield 62.5%; m.p. 161-162° C (dec.) Rf: 0.3;
FD-MS: m/z 586 (MH+), 585 (M+.);
$^1$HNMR (200 MHz, DMSO) inter alia δ:
13.61 (s, 1H, 6-OH), 13.19 (s, 1H, 11-OH), 8.17 (dd, J=1.2, 7.8Hz, 1H, 1-H), 7.99 (dd, J=4.6, 7.8, 8.2Hz, 1H, 2-H), 7.77 (ddd, 1.2, 8.2, 11.7Hz, 1H, 3-H), 5.37 (d, J=3.2Hz, 1H, 1′-H), 5.02 (m, 1H, 7-H), 3.88 (m, 4H, $CH_2OCH_2$), 3.8-3.2 (m, 6H, 4′-H, 3′-H, $CH_2NCH_2$), 2.26 (s, 3H, $COCH_3$), 1.25 (d, J=6.3Hz, 5′-$CH_3$).

Example 4

3′-deamino-3′(2-methoxy-4-morpholinyl)-4′-epi-doxorubicin (j)

To a solution of 0.29 g (0.5 mmole) of 4′-epi-doxorubicin (C1) in 30 ml of acetonitrile-water (1:1) was added 0.65 g (5 mmole) of 1-methoxy-2,2′ oxydiacetaldehyde (D) dissolved in 10 ml of acetonitrile. The pH was adjusted to 7.4 with a solution of sodium hydrogen carbonate. After 30 minutes the mixture was treated with a solution of 0.032 g (0.5 mmole) of sodium cyanoborohydride dissolved in 3 ml of water. After 15 minutes the mixture was worked up by diluting with water (50 ml) and extracted with n-butanol.

The organic phase was evaporated under reduced pressure and the residue was purified by flash

chromatography on silica gel column, using methylene chloride/methanol (97:3 by volume) as eluting system, to give 0.145 g (yield 42%) of the title compound (j) which was isolated as hydrochloride salt after treatment with anhydrous methanolic hydrogen chloride. m.p. 153-154 °C (dec.); Rf: 0.17;
FD-MS: m/z 644 (MH+), 643 (M+.);
$^1$HNMR (200 MHz, CDCl3) inter alia δ:
13.91 (s, 1H, 6-OH), 13.11 (s, 1H, 11-OH), 5.53 (dd, J=3.5, <1Hz, 1H, 1′-H), 5.23 (dd, J=2.0, 3.9Hz, 1H, 7-H), 4.74 (s, 2H, 14-$CH_2OH$), 4.40 (dd, J=2.5, 4.8Hz, 1H,

$OCH_3$
|
$CHeq-CH_2N$),

4.06 (s, 3H, 4-$OCH_3$), 3.5-3.9 (m, 2H, $OCH_2$-$CH_2N$), 3.38 (s, 3H,

$CH_2$
|
$OCH-OCH_3$ ),

3.17 (dd, J=8.7, 8.7Hz, 1H, 4′-H), 2.72 (dd, J=2.5, 11.2Hz, 1H,

$OCH_3$
|
NCHeqCH),

2.5-2.6 (m, 1H, 3′-H), 2.3-2.6 (m, 2H, $NCH_2O$), 2.23 (dd, J=4.8, 11.2HZ, 1H,

$OCH_3$
|
NCHax-CH ),

1.33 (d, J=6.0Hz, 3H, 5′-H)

## Example 5

### 4-demethoxy-3′-deamino-3′(2-methoxy-4-morpholinyl)-daunorubicin (1)

The synthesis of the title compound (1) was performed according to procedure described in Example 4 and starting from 4-demethoxy-daunorubicin (C3).
Yield 40%; m.p. 150-151 °C (with dec.); Rf: 0.25; FD-MS: m/z 627 (M+.)

## Example 6

### 4-demethoxy-6-deoxy-6-amino-3′-deamino-3′(4-morpholino)-doxorubicin (r)

Following the procedure described in Example 1, the title compound (r) was obtained starting from 4-demethoxy-6-deoxy-6-aminodoxorubicin (C15). Yield 45%; m.p. 156-158 °C (with dec.) FD-MS: m/z 584 (M+.)
$^1$HNMR (200 MHz, $CDCl_3$) inter alia δ:
1.11 (d, J=6.6Hz, 3H, 5′-$CH_3$); 2.3-2.6 (m, 8H, $CH_2NCH_2$); 5.02 (dd, J=3.9, 5.3Hz, 1H, 7-H); 5.4 (d, J=2.9

Hz, 1H, 1′-H); 7.7-8.0 (m, 2H, 2-H, 3-H); 8.06 (broad, 2H, $NH_2$); 8.2-8.3 (m, 2H, 1-H, 4-H); 14.2 (1H, 11-OH).

## Claims

1. An anthracycline glycoside of general formula I:

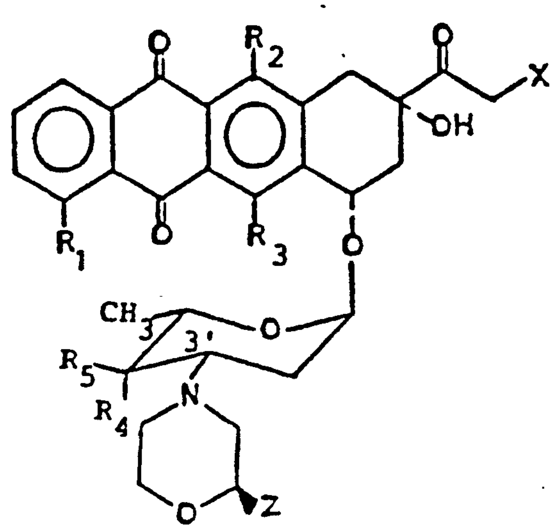

wherein Z is hydrogen or methoxy;
X represents hydrogen or hydroxy;
$R_1$ represents hydrogen, fluorine, hydroxy, methoxy or amino;
$R_2$ and $R_3$ both represent hydroxy or one of $R_2$ and $R_3$ is hydrogen, nitro or amino and the other is hydroxy; and
$R_4$ and $R_5$ represent hydrogen or one of $R_4$ and $R_5$ is hydrogen and the other is hydroxy;
and pharmaceutically acceptable salts thereof; with the provisos that:
(i) when $R_2$ and $R_3$ are both hydroxy and Z is hydrogen then $R_1$ represents hydroxy, fluorine or amino, and
(ii) when both Z and $R_1$ are methoxy and $R_2$ and $R_3$ are both hydroxy then $R_5$ is hydroxy or both $R_4$ and $R_5$ are hydrogen.

2. A compound according to Claim 1, which is selected from 4-demethyl-6-deoxy-3′-deamino-3′-(4-morpholinyl)-doxorubicin or its hydrochloride salt; 4-demethoxy-4-amino-3′-deamino-3′-(4-morpholinyl)-daunorubicin or its hydrochloride salt; 4-demethoxy-4 amino-3′-deamino-3′-(4-morpholinyl)-4′-epi-daunorubicin or its hydrochloride salt; 4-demethoxy-4-fluoro-3′-deamino-3′-(4-morpholinyl)-daunorubicin or its hydrochloride salt; 4-demethoxy-4-fluoro-3′-deamino-3′-(4-morpholinyl)-4′-epi-daunorubicin or its hydrochloride salt; 4-demethoxy-11-deoxy-11-nitro-3′-deamino-3′-(4-morpholinyl)-daunorubicin or its hydrochloride salt; 4-demethoxy-6-deoxy-6-nitro-3′-deamino-3′-(4-morpholinyl)-daunorubicin or its hydrochloride salt; 4-demethoxy-11-deoxy-11-amino-3′-deamino-3′-(4-morpholinyl)-daunorubicin or its hydrochloride salt; 4-demethoxy-6-deoxy-6-amino-3′-deamino-3′-(4-morpholinyl)-daunorubicin or its hydrochloride salt; 3′-deamino-3′-(2-methoxy-4-morpholinyl)-4-epi-doxorubicin or its hydrochloride salt; 3′-deamino-3′-(2-methoxy-4-morpholinyl)-4′-deoxy-doxorubicin or its hydrochloride salt; 4-demethoxy-3′-deamino-3′-(2-methoxy-4-morpholinyl)-daunorubicin or its hydrochloride salt; 4-demethyl-3′-deamino-3′-(2-methoxy-4-morpholinyl)-daunorubicin or its hydrochloride salt; 4-demethyl-6-deoxy-3′-deamino-3′-(2-methoxy-4-morpholinyl)-doxorubicin or its hydrochloride salt; 4-demethoxy-4-amino-3′-deamino-3′-(2-methoxy-4-morpholinyl)-daunorubicin or its hydrochloride salt; 4-demethoxy-11-deoxy-11-amino-3′-(2-methoxy-4- morpholinyl)-daunorubicin or its hydrochloride salt; 4-demethoxy-6-deoxy-6-amino-3′-deamino-3′-(2-methoxy-4-morpholinyl)-daunorubicin or its hydrochloride salt, and 4-demethoxy-6-deoxy-6-amino-3′-deamino-3′-(4-morpholinyl) doxorubicin or its hydrochloride salt.

3. A process for the preparation of an anthracycline glycoside of formula I according to Claim 1 wherein Z = H, or a pharmaceutically acceptable salt thereof, which process comprises reacting an anthracycline glycoside of general formula II:

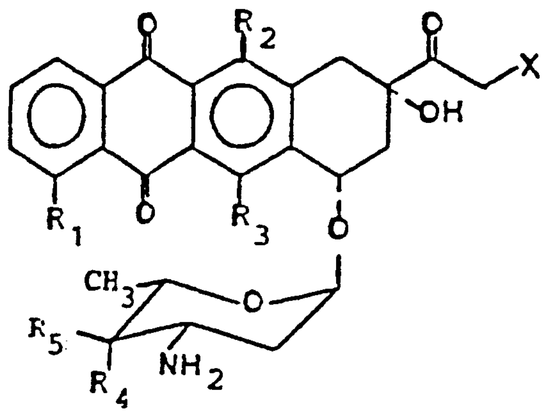

wherein $R_1$, $R_2$, $R_3$, $R_4$, $R_5$ and X are as defined in Claim 1, or a salt thereof, with bis(2-iodoethyl)ether and, if desired, converting the anthracycline glycoside of formula I wherein Z is H thus obtained into a pharmaceutically acceptable salt thereof.

4. A process according to claim 3 wherein the anthracycline glycoside of formula II or salt thereof dissolved in an anhydrous polar solvent is reacted, at room temperature and for 24 hours, with an excess of bis(2-iodoethyl)ether in the presence of triethylamine; the pH of the reaction mixture, previously diluted with water, is adjusted to 7.5 using an aqueous solution of sodium hydrogen carbonate; the reaction mixture is extracted with methylene chloride; the organic solvent is removed under reduced pressure; and, after purification of the crude product on a column of silica gel using a mixture of methylene chloride and methanol (98:2 v/v) as eluting agent, the product is isolated as the hydrochloride salt by addition of a methanolic solution of anhydrous hydrogen chloride.

5. A process for the preparation of an anthracycline glycoside of formula I according to Claim 1 wherein Z = $OCH_3$, or a pharmaceutically acceptable salt thereof, which process comprises effecting reductive alkylation of an anthracycline glycoside of formula II as defined in Claim 3 or a salt thereof using 1-methoxy-2,2′-oxydiacetaldehyde of formula III:

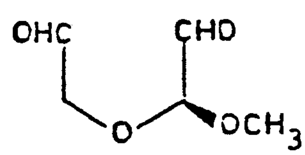

and, if desired, converting the resulting anthracycline glycoside of formula I wherein Z is $OCH_3$ into a pharmaceutically acceptable salt thereof.

6. A process according to Claim 5, wherein the anthracycline glycoside of formula II or salt thereof, dissolved in a mixture of acetonitrile and water (1:1 by volume) is reacted with an excess of the aldehyde of formula III; after 30 minutes added to the reaction mixture, adjusted to pH 7.4, is an aqueous solution of sodium cyanoborohydride; the mixture is extracted with n-butanol; the organic solvent is removed under reduced pressure; and, after purification of the crude product on a column of silica gel using a mixture of methylene chloride-methanol (97:3 v/v) as eluting agent, the desired product is isolated as the hydrochloride salt by addition of a methanolic solution of anhydrous hydrogen chloride.

7. A pharmaceutical composition comprising an anthracycline glycoside of formula I as defined in Claim 1, or a pharmaceutically acceptable acid addition salt thereof, together with a pharmaceutically acceptable carrier or diluent.

8. An anthracycline glycoside of formula I according to Claim 1, or a pharmaceutically acceptable salt thereof, for use as an anti-tumor agent.

Claims for the following Contracting States: ES, GR

1. A process for the preparation of an anthracycline glycoside of formula I

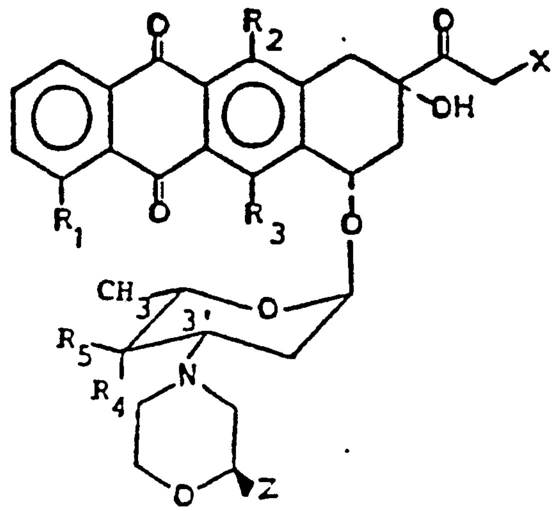

wherein Z = H,
X represents hydrogen or hydroxy;
$R_1$ represents hydrogen, fluorine, hydroxy, methoxy or amino;
$R_2$ and $R_3$ both represent hydroxy or one of $R_2$ and $R_3$ is hydrogen, nitro or amino and the other is hydroxy; and
$R_4$ and $R_5$ represent hydrogen or one of $R_4$ and $R_5$ is hydrogen and the other is hydroxy;
and pharmaceutically acceptable salts thereof; with the provisos that:
(i) when $R_2$ and $R_3$ are both hydroxy and Z is hydrogen then $R_1$ represents hydroxy, fluorine or amino, and
(ii) when both Z and $R_1$ are methoxy and $R_2$ and $R_3$ are both hydroxy then $R_5$ is hydroxy or both $R_4$ and $R_5$ are hydrogen, or a pharmaceutically acceptable salt thereof, which process comprises reacting an anthracycline glycoside of general formula II:

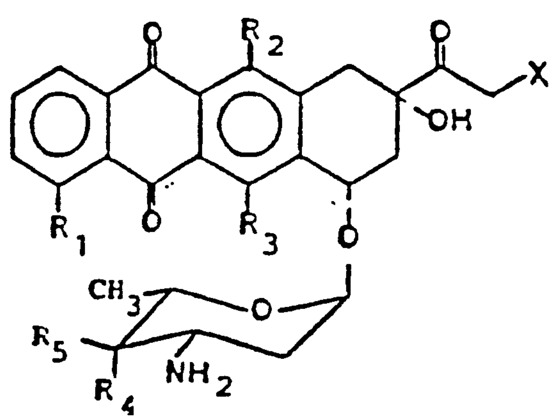

wherein $R_1$, $R_2$, $R_3$, $R_4$, $R_5$ and X are as defined in Claim , or a salt thereof, with bis(2-iodoethyl)ether and, if desired, converting the anthracycline glycoside of formula I wherein Z is H thus obtained into a pharmaceutically acceptable salt thereof.

2. A process according to claim 1 wherein the anthracycline glycoside of formula II or salt thereof dissolved in an anhydrous polar solvent is reacted, at room temperature and for 24 hours, with an excess of bis(2-iodoethyl)ether in the presence of triethylamine; the pH of the reaction mixture, previously diluted with water, is adjusted to 7.5 using an aqueous solution of sodium hydrogen carbonate; the reaction mixture is extracted with methylene chloride; the organic solvent is removed under reduced pressure; and, after purification of the crude product on a column of silica gel using a mixture of methylene chloride and ethanol (98:2 v/v) as eluting agent, the product is isolated as the hydrochloride salt by addition of a methanolic solution of anhydrous hydrogen chloride.

3. A process for the preparation of an anthracycline glycoside of formula I

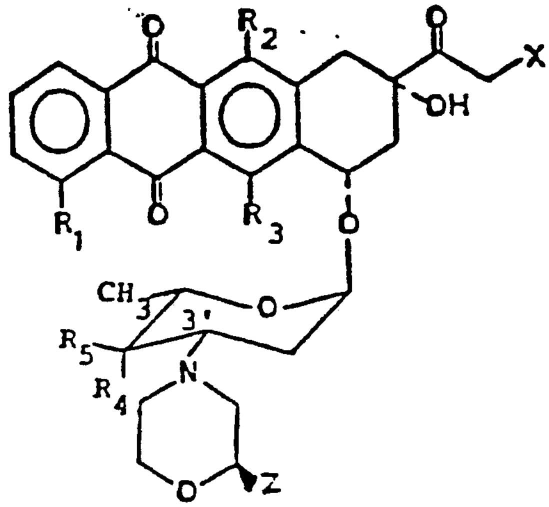

wherein Z = $OCH_3$,
X represents hydrogen or hydroxy;
$R_1$ represents hydrogen, fluorine, hydroxy, methoxy or amino;
$R_2$ and $R_3$ both represent hydroxy or one of $R_2$ and $R_3$ is hydrogen, nitro or amino and the other is hydroxy; and
$R_4$ and $R_5$ represent hydrogen or one of $R_4$ and $R_5$ is hydrogen and the other is hydroxy;
and pharmaceutically acceptable salts thereof; with the provisos that:
(i) when $R_2$ and $R_3$ are both hydroxy and Z is hydrogen then $R_1$ represents hydroxy, fluorine or amino, and
(ii) when both Z and $R_1$ are methoxy and $R_2$ and $R_3$ are both hydroxy then $R_5$ is hydroxy or both $R_4$ and $R_5$ are hydrogen, or a pharmaceutically acceptable salt thereof, which process comprises effecting reductive alkylation of an anthracycline glycoside of formula II as defined in Claim 1 or a salt thereof using 1-methoxy-2,2′-oxydiacetaldehyde of formula III:

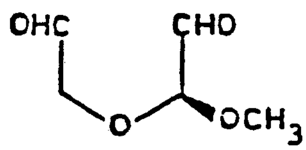

and, if desired, converting the resulting anthracycline glycoside of formula I wherein Z is $OCH_3$ into a pharmaceutically acceptable salt thereof.

4. A process according to Claim 3, wherein the anthracycline glycoside of formula II or salt thereof, dissolved in a mixture of acetonitrile and water (1:1 by volume) is reacted with an excess of the aldehyde of formula III; after 30 minutes added to the reaction mixture, adjusted to pH 7.4, is an aqueous solution of sodium cyanoborohydride; the mixture is extracted with n-butanol; the organic solvent is removed under reduced pressure; and, after purification of the crude product on a column of silica gel using a mixture of methylene chloride-methanol (97:3 v/v) as eluting agent, the desired product is isolated as the hydrochloride salt by addition of a methanolic solution of anhydrous hydrogen chloride.